(19) Europäisches Patentamt
European Patent Office
Office européen des brevets

(11) Veröffentlichungsnummer: **0 183 200**
**B1**

# (12) EUROPÄISCHE PATENTSCHRIFT

(45) Veröffentlichungstag der Patentschrift: **30.01.91**

(51) Int. Cl.$^5$: **C 22 B 3/38,** C 01 G 55/00

(21) Anmeldenummer: **85114832.0**

(22) Anmeldetag: **22.11.85**

## (54) Verfahren zur Rückgewinnung von Rhodium aus Reaktionsprodukten der Oxosynthese.

(30) Priorität: **29.11.84 DE 3443474**

(43) Veröffentlichungstag der Anmeldung:
**04.06.86 Patentblatt 86/23**

(45) Bekanntmachung des Hinweises auf die Patenterteilung:
**30.01.91 Patentblatt 91/05**

(84) Benannte Vertragsstaaten:
**AT BE CH DE FR GB IT LI LU NL SE**

(56) Entgegenhaltungen:
**EP-A-0 147 824**
**EP-A-0 156 253**

(73) Patentinhaber: **HOECHST AKTIENGESELLSCHAFT**
**Postfach 80 03 20**
**D-6230 Frankfurt am Main 80 (DE)**

(72) Erfinder: **Cornils, Boy, Dr. Dipl.-Chem.**
**Friedrich-Ebert-Strasse 45**
**D-4220 Dinslaken (DE)**
Erfinder: **Konkol, Werner, Dr. Dipl.-Chem.**
**Lützwostrasse 40a**
**D-4200 Oberhausen 11 (DE)**
Erfinder: **Bahrmann, Helmut, Dr. Dipl.-Chem.**
**Rohstrasse 42**
**D-4236 Hamminkeln 3 (DE)**
Erfinder: **Bach, Hanswilhelm, Dr. Dipl.-Chem.**
**Alleestrasse 56**
**D-4100 Duisberg 11 (DE)**
Erfinder: **Wiebus, Ernst**
**Ferdinandstrasse 77**
**D-4200 Oberhausen 11 (DE)**

EP 0 183 200 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft ein verbessertes Verfahren zur Abtrennung und Rückgewinnung von Rhodium aus den Reaktionsgemischen de Oxosynthese.

Die Herstellung von Aldehyden und Alkoholen durch Anlagerung von Kohlenmonoxid und Wasserstoff an olefinische Doppelbindungen (Hydroformylierung) ist bekannt. Die Reaktion wird durch Metalle der 8. Nebengruppe des Periodensystems oder deren Verbindungen katalysiert, die unter den Reaktionsbedingungen Carbonyle oder Hydridocarbonyle bilden. Während früher fast ausschließlich Kobalt- und Kobaltverbindungen als Katalysatoren eingesetzt wurden, finden heute in zunehmendem Maße Rhodiumkatalysatoren Anwendung, obgleich Rhodium um ein Vielfaches teurer als Kobalt is. Rhodium gelangt dabei allein oder in Kombination mit Komplexbildnern, z.B. organischen Phosphinen, zum Einsatz. Während die Oxosynthese mit Rhodium als Katalysator Reaktionsdrücke von 250 bis 300 bar ($25 \cdot 10^3$ bis $30 \cdot 10^3$ kPa) erfordert, genügen bei Einsatz von Rhodium-Komplexverbindungen Drücke von 10 bis 50 bar ($1{,}0 \cdot 10^3$ bis $5{,}0 \cdot 10^3$ kPa).

Für Rhodium-Katalysatoren ergeben sich in vielen Fällen deutliche Vorteile. Sie besitzen höhere Aktivität und Selektivität und ermöglichen überdies einen komplikationsfreien Betrieb der Produktionsanlage, insbesondere hinsichtlich der Durchführung der Synthese und der Ausbringung der Produkte aus dem Reaktor. Schließlich kann das klassische Oxoverfahren auf Basis von Kobaltkatalysatoren unter Verwendung der vorhandenen Apparateteile in vielen Fällen mit nur geringen Investitionen auf Rhodiumkatalysatoren umgestellt werden.

Erhetliche Schwierigkeiten bereitet jedoch die annähernd verlustfreie Abtrennung und Wiedergewinnung des Rhodiums, das ohne Komplexbildner als Katalysator eingesetzt wird; es findet sich nach Beendigung der Umsetzung als Carbonylverbindung im Hydroformylierungsprodukt gelöst.

Zur Aufarbeitung wird das Oxorohprodukt üblicherweise mehrstufig entspannt, indem man den Synthesedruck, d.h. etwa 250 bis 300 bar ($25 \cdot 10^3$ bis $30 \cdot 10^3$ kPa), zunächst auf 15 bis 25 bar ($1{,}5 \cdot 10^3$ bis $2{,}5 \cdot 10^3$ kPa) reduziert. Hierbei wird im Rohprodukt gelöstes Synthesegas frei. Anschließend kann man auf Normaldruck entspannen. Vor der Reindarstellung durch Destillation oder der Weiterverarbeitung des Reaktionsproduktes müssen die in ihm homogen gelösten Rhodiumverbindungen entfernt werden. Hierbei ist zu berücksichtigen, daß das Edelmetall im Rohprodukt in einer Konzentration von nur wenigen ppm vorliegt. Zusätzliche Schwierigkeiten können noch dadurch entstehen, daß das Rhodium bei der Entspannung teilweise in metallische Form übergeht oder mehrkernige Carbonyle bildet. In beiden Fällen kommt es dann zur Ausbildung eines heterogenen Systems, das aus der flüssigen organischen Phase und der festen, Rhodium oder Rhodiumverbindungen enthaltenden Phase besteht.

Nach dem Verfahren der deutschen Patentanmeldung P 33 47 406.0 (=EP—A—147824) wird Rhodium dadurch abgetrennt und wiedergewonnen, daß man es mit Hilfe komplexbildender Reagenzien aus dem Oxorohprodukt extrahiert.

Unter dem Begriff Oxorohprodukt wird dabei das nach Entspannen und gegebenenfalls Abkühlen anfallende Reaktionsgemisch de Oxosynthese verstanden.

Nach einer bevorzugten Ausführungsform der älteren Arbeitsweise setzt man als Komplexbildner Sulfonate und Carboxylate organische Phosphine der allgeminen Formel

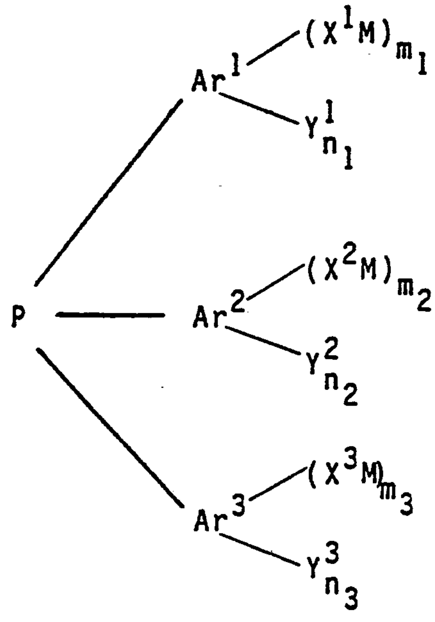

ein. Hierbei bedeuten $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkyoxygruppe, ein Halogenatom, die

OH-, CN-, $NO_2$- oder $R^1R^2N$-Gruppe, in der $R^1$ und $R^2$ für jeweils eine geradkettige oder verzweigte Alkylgruppe mit 1 bis 4 C-Atomen stehen; $X^1$, $X^2$, $X^3$ ist jeweils ein Carboxylat-($COO^-$-) und/oder ein Sulfonat-($SO_3^-$-)Rest, $m_1$, $m_2$, $m_3$ sind gleich oder verschiedene ganze Zahlen von 0 bis 3, wobei mindestens eine Zahl $m_1$, $m_2$, $m_3$ gleich oder größer als 1 ist; $n_1$, $n_2$, $n_3$ sind gleiche oder verschiedene ganze Zahlen von 0 bis 5. M ist ein Alkalimetallion, ein Äquivalent eines Erdalkalimetall-, oder Zinkons, ein Ammonium- oder quaternäres Ammoniumion der allgemeinen Formel $N(R^3R^4R^5R^6)^+$ in der $R^3$, $R^4$, $R^5$, $R^6$ jeweils für eine geradkettige oder verzweigte Alkylgruppe mit 1 bis 4 C-Atomen steht.

Nach einer weiteren bevorzugten Ausführungsform dieses Verfahrens verwendet man als komplexbildende Reagenzien Verbindungen der vorstehend beschriebenen allgemeinen Formel, in der $Ar^1$, $Ar^2$, $Ar^3$ jeweils einen Phenylrest, $X^1$, $X^2$, $X^3$ jeweils einen Sulfonatrest und $m_1$, $m_2$, $m_3$ jeweils die Zahl 1 bedeuten, wobei die Summe von $m_1$, $m_2$ und $m_3$ 1, 2 oder 3 ist.

Die aus Rhodium und den Sulfonaten oder Carboxylaten organischer Phosphine gebildeten Komplexverbindungen sind wasserlöslich. Dementsprechend kann das Rhodium aus dem Oxorohprodukt, also der organischen Phase, mit einer wässrigen Lösung des substituierten Phosphins extrahiert werden. Das Rhodium geht hierbei in die wässrige Phase über, die sich durch einfaches Dekantieren vom organischen Produktgemisch abtrennen läßt. Durch Kreislaufführung der Lösung des Komplexbildners können hohe Rhodium-Konzentrationen in der wässrigen Phase erreicht werden.

Um die Extraktion des Rhodiums aus der organischen Phase und seinen Übergang in die wässrige Phase zu beschleunigen und zu vervollständigen, setzt man nach der deutschen Patentanmeldung P 34 11 034 (=EP—A—156253) der wässrigen Lösung des Komplexbildners einen Lösungsvermittler zu. Seine Wirkung besteht insbesondere darin, daß er die physikalischen Eigenschaften der Grenzflächen zwischen den beiden flüssigen Phasen verändert und dadurch den Übergang des wässrigen Extraktionsmittels in die Produktphase und des Rhodiums aus der Produktphase in die wässrige Komplexbildner-Phase beschleunigt. Durch Mitverwendung eines Lösungsvermittlers wird die Extraktion vereinfacht und der apparative Aufwand verringert.

Die Rhodium-Extraktion ist umso vollständiger, je mehr Lösungsvermittler der wässrigen Phase zugesetzt wird. Seine menge kann jedoch nicht unbegrenzt erhöht werden, weil die zugesetzten Stoffe die wässrige Lösung des Extraktionsmittels unnötig belasten und seine Stabilität beeinträchtigen.

Es ist Aufgabe der vorliegenden Erfindung, diese Nachteile zu beheben.

Sie besteht in einem Verfahren zur Abtrennung und Wiedergewinnung von Rhodium aus den Produkten der Oxosynthese durch Extraktion mit der wässrigen Lösung eines komplexbildenden Phosphins der allgemeinen Formel

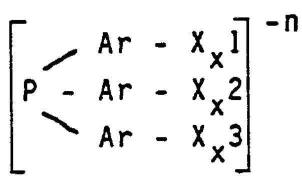
$$[Kat^+]_n$$

wobei Ar eine Arylgruppe, X einen Sulfonatrest ($SO_3$-) und $x^1$, $x^2$, $x^3$ 0 oder 1 bedeuten, mit de Maßgabe, daß mindestens eine Zahl $x^1$, $x^2$ oder $x^3$ 1 ist, n für eine ganze Zahl von 1 bis 3 und $Kat^+$ für ein Kation stehen, Es ist dadurch gekennzeichnete, daß $Kat^+$ der allgemeinen Formel

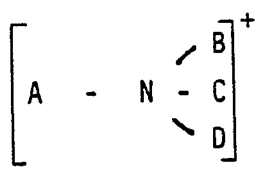

folgt, wobei A ein Alkyl- oder Aralkylrest mit 7 bis 18 Kohlenstoffatomen ist und B, C, D gleich oder verschieden sind und geradkettige oder verzweigte Alkylreste mit 1 bis 4 Kohlenstoffatomen sind.

Die nach dem neuen Verfahren als Extraktionsmittel eingesetzten wasserlöslichen Phosphine (komplexbildende Phosphine oder Reagenzien, Komplexbildner) vermindern offensichtlich die Grenzflächenspannung zwischen der wässrigen Phase und der organischen Phase und erhöhen dadurch die Extraktionsrate. Ihre äußerst geringe Löslichkeit in der organischen Phase hat zur Folge, daß sie und der Rhodiumkatalysator nicht oder in vernachlässigbar kleiner Menge zusammen mit dem Reaktionsprodukt aus der Reaktionszone ausgetragen werden. Ein gesonderter Aufarbeitungsschritt zur Rückgewinning des Rhodiums aus dem Reaktionsprodukt erübrigt sich daher.

Als Extraktionsmittel im Rahmen der neuen Arbeitsweise besonders geeignet sind Phosphine der oben wiedergegebenen allgemeinen Formel, wobei Ar eine Phenyl- oder Naphthylgruppe und B, C und D die gleichen geradkettigen oder verzweigten Alkylgruppen mit 1 bis 4 Kohlenstoffatomen bedeuten oder zusammen mit N für Pyrrol, Pyridin oder Morpholin stehen und die Summe $x^1$, $x^2$ und $x^3$ 2 oder 3 ist.

Beispiele für wasserlösliche Phosphine, die sich zur Durchführung des neuen Verfahrens eignen, sind Triphenyltrisulfonate und Triphenyldisulfonate oder auch deren Gemische mit folgenden Kationen:

Trimethylbenzylammonium, Trimethylcetylammonium, Trimethyldodecylammonium, Tributyldodecylammonium, Dodecylethyl-dimethylammonium, Triethylbenzylammonium.

Zur Herstellung der in dem beanspruchten Verfahren verwendeten Phosphine geht man von sulfonierten Triarylphosphinen aus, die durch Behandlung von Triarylphosphinen mit Oleum erhalten werden. Durch Variation der Reaktionsbedingungen, insbesondere der Reaktionszeit, der Reaktionstemperatur und des Verhältnisses von Triarylphosphin zu Schwefeltrioxid lassen sich mono-, di- oder trisulfonierte Arylphosphine herstellen.

Zweckmäßig gewinnt man aus dem Sulfonierungsprodukt zunächst in Wasser unlösliche, in organischen Lösungsmitteln jedoch lösliche Aminsalze. Sie werden anschließend durch Behandlung mit einem quartären Ammoniumhydroxid in das gewünschte Onium-Slz des sulfonierten Triarylphosphins überführt.

Bezogen auf Rhodium werden die komplexbildenden Phosphine im molaren Überschuß angewendet. Da man sie rezirkulieren kann, ist die Höhe des Überschusses unkritisch, jedoch sollen je g-Atom Rhodium mindestens 5 mol komplexbildendes Phosphin vorhanden sein. Besonders bewährt hat es sich, je g-Atom Rhodium 20 bis 40 mol, bei Rückführung sogar bis 500 mol komplexbildendes Phosphin zu verwenden.

Üblicheweise wird das komplexbildende Phosphin in Form einer Lösung eingesetzt. Hierbei ist darauf zu achten, daß es ebenso wie der entstehende Rhodiumkomplex, im Reaktionsprodukt weitgehend unlöslich, dagegen im Lösungsmittel für das komplexbildende Reagenz gut löslich ist. Selbstverständlich darf auch das Lösungsmittel mit dem Reaktionsprodukt nicht oder nur sehr wenig mischbar sein.

Bei Erfüllung dieser Bedingungen bildet sich ein Zweiphasensystem aus, das aus dem Reaktionsprodukt und der Lösung des komplexbildenden Phosphins bzw. der entstandenen Rhodium-Komplexverbindung besteht. Der Komplexbildner wirkt als Extraktionsmittel, d.h. im Anfangszustand befindet sich das Rhodium im Reaktionsprodukt gelöst, im Endzustand in der Lösung des Komplexbildners. Die Trennung von Reaktionsprodukt und Rhodium enthaltender Lösung erfolgt einfach durch Dekantieren der beiden Phasen voneinander.

Das bevorzugte Lösungsmittel für das komplexbildende Reagenz und den Rhodiumkomplex ist Wasser. Es können auch andere Lösungsmittel verwendet werden, sofern sichergestellt ist, daß Oxoprodukt und Lösungsmittel sich miteinander nicht mischen.

Die Konzentration des komplexbildenden Reagenzes im Lösungsmittel ist in weiten Grenzen variabel. Sie hängt insbesondere davon ab, in welchem Maße das Rhodium angereichert werden soll. Dementsprechend können nicht nur stark verdünnte, sondern sogar gesättigte Lösungen Anwendung finden. In der Regel setzt man Lösungen ein, die 0,5 bis 75 Gew.% und vorzugsweise 1 bis 10 Gew.% (jeweils bezogen auf die Lösung) des Komplexbildners enthalten.

Sofern der Komplexbildner unter den Extraktionsbedingungen flüssig und im Hydroformylierungsprodukt unlöslich oder schwerlöslich und weiterhin der Rhodiumkomplex im komplexbildenden Phosphin löslich ist, kann auf die Mitverwendung eines Lösungsmittels auch verzichtet, also der reine Komplexbildner eingesetzt werden.

Die Extraktion des Rhodiums mit dem gelösten oder reinen Komplexbildner wird bei Temperaturen von 0 bis 200°C, vor zugsweise 20 bis 100°C durchgeführt. In einzelnen Fällen hat sich aber auch bewährt, bei 120 bis 150°C zu arbeiten.

Der neue Prozeß kann absatzweise oder kontinuierlich durchgeführt werden. Schon bei einfacher Rückführung des teilweise beladenen Komplexbildners in das Reaktionsprodukt lassen sich hohe Rhodiumkonzentrationen im Extraktionsmittel erreichen. In einer anderen Variante des erfindungsgemäßen Verfahrens erfolgt die Extraktion mehrstufig, wobei das rhodiumhaltige Reaktionsprodukt im Gegenstrom zum Extraktionsmittel geführt wird.

Nach einer bevorzugten Variante des erfindungsgemäßen Verfahrens wird die vom Rhodium befreite organische Phase zur Enternung restlichen Extraktionsmittels und Rhodiums mit Wasser gewaschen und danach destilliert. Das Waschwasser kann im Kreis geführt werden. Ein Teilstrom kann zur Ergänzung von Wasserverlusten in die Extraktionsstufe geleitet werden, die dadurch auftreten, daß das Rohprodukt laufend geringe Mengen Wasser aus der Extraktionsmittel-Lösung entnimmt. Diese Wassermenge wird in der Waschstufe als Frischwasserzusatz ergänzt.

Die Weiterbehandlung oder Weiterverwendung der das abgetrennte Rhodium enthaltenden Phase richtet sich nach den jeweiligen Gegebenheiten. So kann das Rhodium in bekannter Weise, z.B. durch Überführung in das Salz einer höheren Carbonsäure abgetrennt und wiederum als Katalysator eingesetzt werden. Es ist aber auch möglich, die aus Lösungsmittel, Rhodium und Komplexbildner bestehende Phase unmittelbar als Katalysatorsystem zu verwenden.

Mit besonderem Erfolg wird das erfindungsgemäße Verfahren zur Abtrennung und Wiedergewinnung von Rhodium aus den Produkten der Hydroformylierung end- und innenständiger verzweigter Olefine mit mehr als 5 Kohlenstoffatomen wie i-Hepten, Diisobutylen, Tri- und Tetrapropylen oder dem unter der Bezeichnung Dimersol im Handel befindlichen Gemisch von $C_8$-Olefinen angewandt. Selbstverständlich kann das Verfahren auch auf die Hydroformylierung unverzweigter end- und mittelständiger Olefine angewendet werden, wobei in der Regel die absoluten Rh-Konzentrationen niedriger liegen.

In den nachstehenden Beispielen werden verschiedene Ausführungsformen der Erfindung beschrieben, das beanspruchte Verfahren ist jedoch nicht auf diese Ausführungsformen beschränkt.

In den Beispielen 1 bis 8 wird jeweils auf 20 bis 25°C abgekühlter und mehrere Stunden gelagerter

roher Isooctylaldehyd, erhalten durch Hydroformylierung von i-Hepten, eingesetzt. Die Beispiele 1, 3, 5, 7 betreffen die Rhodium-Extraktion mit dem TPPTS-Na-Salz als Komplexbildner; in den Beispielen 2, 4, 6, 8 wird ein TPPTS-Onium-Salz verwendet. In den Beispielen 9 und 10 wird die Abtrennung von Rhodium aus verschiedenen Hydroformylierungsprodukten beschrieben, wobei in dem Beispiel 9 mit TPPTS-Na-Salz gearbeitet wird.

Die Abkürzung TPPTS steht für Triphenylphosphintrisulfonat. Alle Konzentrationsangaben erfolgen in Gew.%.

Beispiel 1 (Vergleich)

In einem Rührkolben werden 200 g roher Isooctylaldehyd, der

34,9% $C_7$-Kohlenwasserstoff (überwiegend Hepten)
62,7% Isooctylaldehyd
2,2% Isooctylalkohol
0,2% Höhersieder

und 3,9 ppm Rhodium enthält mit 20 g einer 0,1-prozentigen wässrigen Natrium-TPPTS-Lösung versetzt. Das molare Verhältnis von Phosphor zu Rhodium beträgt 5. Beide Phasen werden 5 Minuten bei 50°C intensiv gerührt. Nach Beeindigung des Rührens trennen sich die beiden Phasen innerhalb von 12 Sekunden ohne Emulsionsbildung. Die organische Oxorohproduktphase enthält noch 1,1 ppm Rhodium, entsprechend einer Rhodiumabtrennung von 72%.

Beispiel 2

Es wird wie in Beispiel 1 verfahren mit dem Unterschied, daß eine wässrige TPPTS-Benzyltrimethylammonium-Salz-Lösung eingesetzt und eine Minute gerührt wird. Die organische Oxorohprodukt-Phase enthält lediglich noch 0,5 ppm Rhodium, entsprechend einer Rhodiumabtrennung von 87%.

Beispiel 3 (Vergleich)

Es wird wie in Beispiel 1 gearbeitet, jedoch unter Verwendung einer 0,4-prozentigen Natrium-TPPTS-Lösung. Das molare Verhältnis von Phosphor zu Rhodium beträgt 20. In der organischen Phase bleibt 1 ppm Rhodium zurück, entsprechend einer Rhodiumabtrennung von 74%.

Beispiel 4

Es wird wie in Beispiel 3 gearbeitet mit dem Unterschied, daß eine wässrige TPPTS-Benzyltriethylammonium-Salz-Lösung eingesetzt und eine Minute gerührt wird. In der organischen Phase verbleiben 0,5 ppm Rhodium, entsprechend einer Rhodiumabtrennung von 87%.

Beispiel 5 (Verleich)

Es wird wie in Beispiel 3 gearbeitet, jedoch bei einer Temperatur von 80°C (statt 50°C) gerührt. Im Rohprodukt bleibt 1 ppm Rhodium zurück, entsprechend einer Rhodiumabtrennung von 74%.

Beispiel 6

Es wird wie in Beispiel 5 gearbeitet, jedoch unter Verwendung von Dodecylethyldimethylammonium-TPPTS-Salz und einer Rührzeit von einer Minute. Im Rohprodukt bleiben 0,4 ppm Rhodium zurück, entsprechend einer Rhodiumabtrennung von 90%.

Beispiel 7 (Vergleich)

In einem Rundkolben mit Bodenablaß, Gaseinleitungskapilare und Rührer werden 1 000 g Isooctylaldehyd de in Beispiel 1 genannten Zusammensetzung mit jeweils 100 g 20-prozentiger Natrium-TPPTS-Lösung extrahiert. Über die Gaseinleitungskapillare wird zunächst Synthesegas ($CO/H_2 = 1:1$) zur Sättigung der Mischung mit CO und Wasserstoff eingeleitet und anschließend bei 80°C intensiv gerührt. Die Rührzeit sowie die anschließende Ruhezeit betragen 30 Sekunden. Darauf wird die wässrige Phase über den Bodenablaß ausgeschleust un die organische Phase erneut mit 100 g einer 20-prozentigen Natrium-TPPTS-Lösung behandelt. Insgesamt wird viermal extrahiert. Die organische Phase enthält danach nur noch 0,6 ppm Rhodium, entsprechend einer Rhodiumabtrennung von 85%.

Beispiel 8

Es wird entsprechend Beispiel 7 gearbeitet mit dem Unterschied, daß die Extraktionsschritte mit einer Lösung des TPPTS-Benzyltrimethylammonium-Salzes erfolgen und die Rührzeit nur 20 Sekunden beträgt. Nach Abschluß der Extraktionen weist die organische Phase noch 0,2 ppm Rhodium auf, entsprechend einer Rhodiumabtrennung von 95%.

Beispiel 9 (Vergleich)

In der Apparatur des Beispiels 7 werden 1 000 g roher Propionaldehyd, der

96,3% Propionaldehyd
0,2% n-Propanol
1,4% Ethylen + Ethan
2,1% Höhersieder

und 9,6 ppm Rhodium enthält mit jeweils 100 g 20-prozentiger Natrium-TPPTS-Lösung in 5 Extraktionsschrittgen bei 36°C behandelt. Der Rhodiumgehalt der organischen Phase beträgt nach de 1. Extraktion 1 ppm, entsprechend einer Rhodiumabtrennung von 89,6% und nach der 5. Extraktion 0,6 ppm, entsprechend einer Rhodiumabtrennung von 94%.

Beispiel 10

Es wird wie in Beispiel 9 gearbeitet mit dem Unterschied, daß eine wässrige TPPTS-Dodecylethyldimethylammonium-Salz-Lösung zugesetzt wird. Der Rhodiumgehalt der organischen Phase nach der 1. Extraktion beträgt 0,5 ppm, entsprechend einer Rhodiumabtrennung von 95% und nach der 5. Extraktion 0,1 ppm, entsprechend einer Rhodiumabtrennung von praktisch 100%.

Entsprechend verbesserte Ergebnisse erhält man auch bei kontinuierliche Extraktion.

**Patentansprüche**

1. Verfahren zur Abtrennung und Wiedergewinnung von Rhodium aus den Produkten der Oxosynthese durch Extraktion mit de wässrigen Lösung eines komplexbildenden Phosphins de allgemeinen Formel

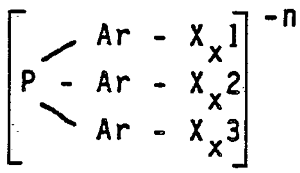

$\left[Kat^+\right]_n$

wobei Ar eine Arylgruppe, X einen Sulfonatrest ($SO_3$-) und $x^1$, $x^2$, $x^3$ 0 oder 1 bedeuten, mit der Maßgabe, daß mindestens eine Zahl $x^1$, $x^2$ oder $x^3$ 1 ist, n für eine ganze Zahl von 1 bis 3 und $Kat^+$ für ein Kation stehen, dadurch gekennzeichnet, daß $Kat^+$ der allgemeinen Formel

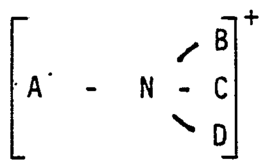

folgt, wobei A ein Alkyl- ode Aralkylrest mit 7 bis 18 Kohlenstoffatomen ist und B, C, D gleich oder verschieden sind und geradkettige oder verzweigte Alkylreste mit 1 bis 4 Kohlenstoffatomen sind.

2. Verfahren nach Anspruch 1, dadurch gekennzeichnet, daß Ar eine Phenyl- oder Naphthylgruppe B, C, D die gleichen geradkettigen oder verzweigten Alkylgruppen mit 1 bis 4 Kohlenstoffatomen bedeuten oder zusammen mit für Pyrrol, Pyridin oder Morpholin stehen und die Summe $x^1$, $x^2$ und $x^3$ 2 oder 3 ist.

3. Verfahren nach Anspruch 1 bis 2, dadurch gekennzeichnet, daß je g-Atom Rhodium mindestens 5 mol komplexbildendes Phosphin vorhanden sind.

4. Verfahren nach Anspruch 3, dadurch gekennzeichnet, daß je g-Atom Rhodium 20 bis 40 mol komplexbildendes Phosphin vorhanden sind.

5. Verfahren nach Anspruch 1 bis 4, dadurch gekennzeichnet, daß das komplexbildende Phosphin als Lösung in Wasser in einer Konzentration von 0,5 bis 75 Gew.%, vorzugsweise 1,0 bis 10 Gew.% (jeweils bezogen auf die Lösung) eingesetzt wird.

6. Verfahren nach Anspruch 1 bis 5, dadurch gekennzeichnet, daß die Extraktion bei 20 bis 100°C durchgeführt wird.

**Revendications**

1. Procédé pour la séparation et la récupération de rhodium à partir des produits de l'oxosynthèse par extraction par la solution aqueuse d'une phosphine complexante de formule générale

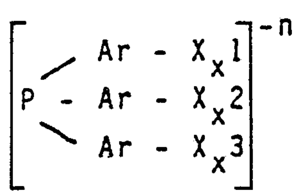

$\left[Cat^+\right]_n$

dans laquelle Ar représente un groupe aryle, X représente un reste sulfonate ($SO_3^-$) et $x^1$, $x^2$ et $x^3$ représentent 0 ou 1, avec la condition que l'un au moins des nombres $x^1$, $x^2$ et $x^3$ est égal à 1, n représente un nombre entier de 1 à 3 et $Cat^+$ représent un cation, caractérise en ce que $Cat^+$ répond à la formule générale

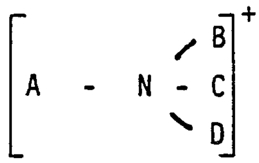

dans laquelle A est un reste alkyle ou arylalkyle en $C_7$—$C_{18}$ et B, C et D sont identiques ou différents et représentent des restes alkyles à chaîne droite ou ramifiée en $C_1$—$C_4$.

2. Procédé selon la revendication 1, caractérisé en ce que Ar représente un groupe phényle ou naphtyle, B, C et D représentent les mêmes groupes alkyles à chaîne droite ou ramifiée en $C_1$—$C_4$ ou bien avec l'atome d'azote un noyau pyrrole, pyridine ou morpholine et la somme de $x^1$, $x^2$ et $x^3$ est égale à 2 ou 3.

3. Procédé selon les revendications 1 et 2, caractérisé en ce qu'il a au moins 5 mol de phosphine complexante par atome-gramme de rhodium.

4. Procédé selon la revendication 3, caractérisé en ce qu'il y a 20 à 40 mol de phosphine complexante par atome-gramme de rhodium.

5. Procédé selon les revendications 1 à 4, caractérisé en ce que la phosphine complexant est utilisée en solution dans l'eau à une concentration de 0,5 à 75% en poids, de préférence de 1,0 à 10% en poids (chaque fois par rapport à la solution.

6. Procédé selon les revendications 1 à 5, caractérisé en ce que l'extraction est mise en oeuvre à 20—100°C.

**Claims**

1. A process for the separation and recovery of rhodium from the products of the Oxo synthesis by extraction with the aqueous solution of a complexing phosphine having the general formula

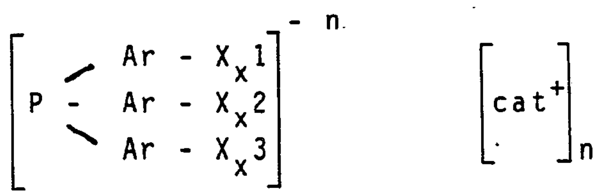

where Ar stands for an aryl group, X a sulfonate radical ($SO_3$-) and $x^1$, $x^2$ and $x^3$ denote 0 or 1, subject to the proviso that at least one number $x^1$, $x^2$ or $x^3$ is 1, n stands for an integer from 1 to 3 and $cat^+$ for a cation, characterised in that $cat^+$ has the general formula

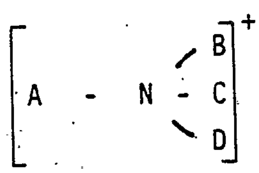

where A is an alkyl or aralkyl radical having 7 to 18 carbon atoms and B, C, and D are the same or different and are straight-chain or branched alkyl radicals having 1 to 4 carbon atoms.

2. A process according to claim 1, characterised in that Ar denotes a phenyl or naphthyl group, B, C and D stand for the same straight-chain or branched alkyl groups having 1 to 4 carbon atoms or together with N stand for pyrrole, pyridine or morpholine and the sum of $x^1$, $x^2$ and $x^3$ is 2 or 3.

3. A process according to claims 1 to 2, characterised in that at least 5 mol of complexing phosphine are present per gramme-atom of rhodium.

4. A process according to claim 3, characterised in that 20 to 40 mol of complexing phosphine are present per gramme-atom of rhodium.

5. A process according to claims 1 to 4, characterised in that the complexing phosphine is used as a solution in water in a concentration of 0.5 to 75% by weight, preferably 1.0 to 10% by weight (in each case related to the solution).

6. A process according to claims 1 to 5, characterised in that extraction is performed at 20 to 100°C.